# EUROPÄISCHE PATENTSCHRIFT

(11) **EP 2 283 887 B1**
(45) Veröffentlichungstag und Bekanntmachung des Hinweises auf die Patenterteilung: **04.07.2012**
(21) Anmeldenummer: 09167361.6
(22) Anmeldetag: 06.08.2009
(51) Int. Cl.: A61M 15/00

(54) **Vorrichtung zur Flussbegrenzung bei niedrigen Differenzdrücken**
Device for flow restriction at low differential pressures
Dispositif de limitation de flux à basses pressions différentielles

(43) Veröffentlichungstag der Anmeldung: 16.02.2011
(73) Patentinhaber: Activaero GmbH, 35285 Gemünden (DE)
(72) Erfinder: Huber, Martin, 82281 Egenhofen (DE); Kolb, Tobias, 80687 München (DE); Müllinger, Bernhard, 80634 München (DE)
(74) Vertreter: Vossius & Partner

(56) Entgegenhaltungen:
- WO-A2-03/000329
- WO-A2-2008/016698
- DE-A1- 10 029 119
- GB-A- 2 312 848
- US-A- 5 655 520
- US-A- 5 727 546
- US-B1- 6 606 992

## Beschreibung

Die Erfindung betrifft eine Vorrichtung zur Flussbegrenzung bei niedrigen Differenzdrücken, insbesondere zur Begrenzung des Inhalationsvolumenstroms bei der Inhalation von therapeutischen Aerosolen bzw. von dosierten Arzneistoffen in Aerosolform in die Lunge, oder in- oder exhalierten Atemgasen. Geeignete Arzneistoffe umfassen Analgetika, Antianginamittel, Antiallergica, Antihistamine und entzündungshemmende Mittel, Expectorantia, Antitussiva, Bronchodilatoren, Diuretika, Anticholingerica, Corticoide, Xanthine, Antitumormittel, therapeutisch wirksame Proteine oder Peptide wie Insulin oder Interferon, Antioxidanzien, antiinflamatorische Substanzen, Wirkstoffe bzw. Medikamente sowie Kombinationen davon.

Bevorzugt ist die Verabreichung von Arzneistoffen zur Behandlung von respiratorischen Erkrankungen wie Asthma, sowie Mittel zur Prophylaxe und Behandlung der Schleimhäute des Tracheo-Bronchialtraktes. Hier ist die Verabreichung von Kortikoiden möglich.

Weiter bevorzugtes Anwendungsgebiet ist die variable Flussbegrenzung bei Lungendiagnosegeräten. Dies ist denkbar bei allen Messverfahren, welche zum Beispiel Aerosolteilchen für die Diagnose nützen.

Die DE-A-199 12 461 offenbart eine Vorrichtung zur Flussbegrenzung bei niedrigen Differenzdrücken, insbesondere zur Begrenzung des Inhalationsvolumenstroms bei einer Inhalation von therapeutischen Aerosolen. Die Vorrichtung besteht aus einem Gehäuse mit einer Einsaugöffnung, einer Aussaugöffnung und einem zwischen diesem angeordneten Strömungskanal mit einem flachen, länglichen Querschnitt mit flexiblen großflächigen Wänden. Der Querschnitt des Strömungskanals ist abhängig von dem Differenzdruck zwischen Aussaugöffnung und Einsaugöffnung sowie der Materialflexibilität des Wandmaterials zu einer Größe für einen vorgegebenen maximalen Inhalationsvolumenstrom verringerbar.

Die Verabreichung von Arzneistoffen in Aerosolform durch Inhalation in die Lunge ist im wesentlichen durch vier Faktoren beeinflusst: (i) die Partikelgröße und die Partikeleigenschaften des Aerosols; (ii) das Atemzugvolumen des Patienten; (iii) der Atemfluss des Patienten; und (iv) die Morphometrie und Atemwege des Patienten. Während bisherige Systeme zwar Aerosole in geeigneten Teilchengrößenbereichen produzieren, werden bei bekannten Systemen die Parameter "Atemzugvolumen" und "Atemfluss" (Atemgeschwindigkeit) nur ungenügend oder gar nicht berücksichtigt. Dies führt zu einer unkontrollierten Inhalation des Aerosols, was wiederum dazu führt, dass eine unzureichende Menge an Aerosolteilchen die Lunge erreicht bzw. innerhalb der Lunge die zu therapierenden Bereiche (beispielsweise Alveolarbereich) nicht erreicht.

In der EPA-0 965 355 wird eine Vorrichtung zum gesteuerten inhalatorischen Einbringen von Dosiermedikamenten in die Lunge vorgeschlagen. Dieser gesteuerte Inhalator weist einen geschlossenen Behälter auf, der mit einem vorbestimmbaren Aerosolvolumen befüllbar ist und aus dem das Aerosol mittels einer Steuereinrichtung für den Inhalationsfluss entnehmbar ist. Bei diesem bekannten Inhalator ist diese Steuereinrichtung entweder ein Verstellventil oder eine kritische Düse. Durch die Verwendung eines verstellbaren Ventils oder einer kritischen Düse kann eine Atemflusslimitierung erreicht werden.

In der EP-B-0 050 654 wird ein Atemgerät zur medikamentösen Behandlung der Lunge vorgeschlagen. Dieses Atemgerät weist eine aufblasbare Hülle auf, aus der mittels eines Mundstückes Aerosol inhaliert werden kann. Dieses Aerosol wir vor der Inhalation aus einer Kartusche über einen Zerstäuber in die aufblasbare Hülle eingebracht. Zur Begrenzung der Durchflussmenge der Luft durch das Mundstück bei der Inhalation weist das Mundstück eine Verengung auf. Diese Verengung limitiert den Atemfluss bei der Inhalation.

Die beiden genannten Inhalationsvorrichtungen zeichnen sich dadurch aus, dass eine FlussLimitierung erfolgt, d. h. während der Inspirationsphase steigt der Atemfluss lediglich langsam an und der Anstieg des Atemflusses nimmt stetig ab, was bei graphischer Darstellung des Atemflusses gegenüber der Zeit zu einer ständigen Abflachung der Kurve führt. Diese Flusslimitierung führt dazu, dass je nach Saugvermögen des Patienten der Atemfluss unterschiedlich bis auf einen maximalen Flusswert ansteigt. Der Fluss wird so nahezu konstant gehalten. Dies bedeutet, dass bei den bekannten Inhalatoren die vorgesehene Flusslimitierung zu einer konstanteren Aerosoldeposition in der Lunge führen kann.

Die EP-A-1 036 569 offenbart ein Verfahren und eine Vorrichtung zur Bereitstellung einer konstanten Medikamenten-Dosis für eine inhalatorische Applikation mit niedrigem Inhalationsfluss. Diese Vorrichtung besteht aus einem volumenreduzierbaren, geschlossenen Behälter, einen mit dem Behälter verbundenen Mundstück, an dem zur Aerosol-Bereitstellung ein Pulver-Aerosol-Inhalator anschließbar ist, einem den Behälter geschlossen umgebenes, volumenreduzierbares Gehäuse, aus dem das Mundstück abgedichtet herausgeführt ist, und aus einer Einrichtung zur Steuerung des Ein- und Auslassens von Luft in den bzw. aus dem Bereich zwischen dem Behälter und Gehäuse. Das Gehäuse ist aus einem volumenreduzierten Zustand in einen vorgesehenen, expandierten Bereitstellungszustand zur Befüllung des Behälters mit dem vorgesehenen Aerosolvolumen bringbar.

Des weiteren offenbart die DE-A-100 29 119 eine Vorrichtung zur Flussbegrenzung bei niedrigen Differenzdrücken, insbesondere für das Begrenzen des Inhalationsvolumenstroms bei der Inhalation von therapeutischen Aerosolen. Diese Vorrichtung besteht aus einem Gehäuse mit wenigstens einer Einsaugöffnung, wenigstens einer Aussaugöffnung und einem zwischen diesen angeordneten Strömungsbereich mit wenigstens einer flexiblen Wand, deren Querschnitt abhängig von dem zwischen Ansaugöffnung und Einsaugöffnung herrschenden Unterdruck und der Materialflexibilität des Wandmaterials bis zu einer vorbestimmten Größe für einen vorgegebenen maximalen Inhalationsvolumenstrom verringerbar ist.

Die EP-A-1 136 921 offenbart eine Inhalationsvorrichtung mit einem selbst expandierbaren Behältnis für ein vorbestimmtes Aerosolvolumen, einer Einrichtung zum Einbringen von Aerosol aus einem Aerosolspender in das Behältnis und einer Steuereinrichtung zum Steuern des Inhalationsflusses. Die Steuereinrichtung hält den Inhalationsfluss während der gesamten Inhalation des Aerosols im wesentlichen konstant, wobei die Steuereinrichtung vier zwischen einer zentralen Einlassöffnung und zu der Einlassöffnung radial beabstandeten Auslassöffnungen radial verlaufende Strömungskanäle aufweist. Die vier radial verlaufenden Strömungskanäle sind durch vier sternförmig angeordnete, rechteckige Stege gebildet, die sich von einer im Wesentlichen starren Wand zu einer im Wesentlichen flexiblen Wand erstrecken. Dabei ist ein Steg länger als die anderen.

Im Bereich der Aerosoltherapie ist es für die vorgesehene Verabreichung des Medikaments wichtig, dass ein bestimmter Inhalationsvolumenstrom nicht überschritten wird. Gleichzeitig soll der Patient möglichst wenig Atemarbeit am Inhalationsgerät verrichten. Das heißt während der Inspiration soll der Patient keinen großen Unterdruck aufbauen müssen, damit die Inhalation auch von Patienten mit schlechter Lungenfunktion durchgeführt werden kann. Um die Mobilität der Patienten zu gewährleisten müssen besonders Inhalatoren zur Verabreichung von Notfallmedikamenten wie z.B. schnell wirkende Betha-2-Sympathomimetika mit kleinen handlichen Inhalationsgeräten verabreicht werden. Mit dem bisherigen Stand der Technik war es jedoch nicht möglich, eine Atemflusskontrolle aufgrund der großen Abmaße der Flusslimitierungsventile in Handgeräte zu integrieren. Gängige Dosieraerosol-Inhalationssysteme, sei es für Flüssig- oder Trockenpulveraerosole, weisen eine kompakte meist mit einer Hand bedienbare Bauform auf. Derartige Inhalationssysteme besitzen keinerlei Vorrichtung um den negativen Effekt eines zu hohen Luftstroms auf eine gute Wirkstoffdeposition zu verhindern. Eine bestimmungsgemäße Volumenstrombegrenzung während der Inhalation von therapeutischen Aerosolen kann in Handgeräten nicht erreicht werden.

Der vorliegenden Erfindung liegt die Aufgabe zugrunde, eine miniaturisierte Vorrichtung zur Flussbegrenzung bei niedrigen Differenzdrücken bei zugleich gleichbleibenden oder sogar verbesserten Funktionsparametem für den Einsatz in Handgeräten bereitzustellen. Diese Aufgabe wird durch Vorrichtung mit den Merkmalen der Ansprüche gelöst.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem ersten Aspekt weist ein Gehäuse mit mindestens einer Einlassöffnung, mindestens einer Auslassöffnung einem dazwischen befindlichen Strömungskanal auf. Der Strömungskanal ist durch eine sich entlang des Strömungskanals erstreckende flexible Wand begrenzt. Ferner weist die flexible Wand eine regelnde Fläche A von weniger als 100mm² auf. Unter regelnder Fläche A wird erfindungsgemäß diejenige Fläche bzw. Teilfläche der flexiblen Wand verstanden, die einen Beitrag zur Regelstrecke des Flussbegrenzers leistet. Dies ist die Grundfläche des variablen Strömungskanals, verringert um die Fläche der Einlassöffnung und der Auslassöffnung. Dies ist die Fläche des Strömungskanals, die in die Regelung eingreift, also die "aktive" Fläche. Vorzugsweise ist die regelnde Fläche A kleiner als 75mm², mehr bevorzugt kleiner als 15 mm².

Der Strömungskanal weist in Strömungsrichtung gesehen einen flachen länglichen Querschnitt a x b auf, der durch die flexible Wand, einer der flexiblen Wand gegenüberliegende Wand, und zwei Querwände gebildet ist. Diese Querschnittsfläche beträgt dabei vorzugsweise weniger als 15 mm². Die Höhe b des Strömungskanals ist maximal 3 mm vorzugsweise weniger als 2 mm. In der Ruhe bzw. Ausgangsstellung ist der Querschnitt a x b über die Länge des Strömungskanals konstant. Durch den bei einer Inhalation des Aerosols entstehenden Unterdruck im Strömungskanal reduziert die mindestens eine flexible Wand den Querschnitt des Strömungskanals.. Dadurch entsteht kein Staudruck bzw. keine Entspannung des Drucks. Vorzugsweise ist die erfindungsgemäße Flussbegrenzungsvorrichtung derart ausgestaltet, dass zum Erreichen eines Gasdurchflusses von maximal 30 l/min, vorzugsweise 12 l/min, ein Differenzdruck von weniger als 30 mbar vorzugsweise weniger als 10 mbar, je nach Größe des Strömungskanals, benötigt wird.

Der kürzeste Abstand zwischen Einlassöffnung und Auslassöffnung ist erfindungsgemäß kleiner als 5 mm, mehr bevorzugt etwa 1,5 mm. Die gesamte Flussbegrenzungsvorrichtung weist vorzugsweise eine Gesamtlänge außen von weniger als 25 mm, vorzugsweise weniger als 22 mm auf, und ist höchstens 15 mm, vorzugsweise höchstens 12 mm breit. Die gesamte Höhe beträgt vorzugsweise höchstens 7 mm, vorzugsweise 4 mm. Bei derartigen Abmessungen wird erfindungsgemäß ein Inhalationsfluss von 30 l/min bei einem am Mundstück der Inhalationsvorrichtung anliegendem Differenzdruck von weniger als 30 mbar erreicht. Vorzugsweise ist auch ein Fluss von 12 l/min bei weniger als 10 mbar, je nach Größe des Strömungskanals, möglich.

Ein weiteres bevorzugtes kennzeichnendes Merkmal der erfindungsgemäßen Vorrichtung ist das Verhältnis von regelnder Fläche A des Strömungskanals zu Querschnittsumfang (entsprechend 2 x a + 2 x b) des regelnden bzw. aktiven Strömungskanals in Ruhestellung. Dieses Verhältnis ist vorzugsweise kleiner als 2, mehr bevorzugt kleiner als 1,4. Ein weiteres bevorzugtes kennzeichnendes Merkmal der erfindungsgemäßen Vorrichtung ist das Verhältnis von regelnder Fläche A des Strömungskanals zu Umfang U der regelnden Fläche in Ruhestellung. Dieses Verhältnis ist vorzugsweise kleiner als 2, mehr bevorzugt kleiner als 1 und meist bevorzugt kleiner 0,7. Ein Verhältnis in diesem Bereich ermöglicht einen niedrigen Druckabfall im Strömungskanal. Damit unterscheidet sich die Erfindung deutlich vom Stand der Technik durch seine Verringerung der Länge und Breite der Grundfläche des variablen Strömungskanals bei zugleich deutlich verringertem Fläche-Umfang-Verhältnis.

Ferner ist gemäß einer bevorzugten Ausführungsform das Verhältnis zwischen Querschnittsfläche von Einsaug- oder Aussaugöffnung zur regelnden Fläche A der flexiblen Wand kleiner 5 zu 1, vorzugsweise kleiner 3 zu 1, und größer 1 zu 1 ist.

Ferner ist gemäß einer bevorzugten Ausführungsform das Verhältnis von regelnder Fläche A des Strömungskanals zu Querschnitt (a x b) Strömungskanal in Ruhestellung, d.h. ohne anliegenden Differenzdruck, kleiner 3, vorzugsweise kleiner 2.

Das Flussbegrenzungsverhalten der erfindungsgemäßen Vorrichtung weist bis zu einem Differenzdruck von 30 mbar eine Hysteresekurve auf, die bei fallenden Differenzdruckwerten höchstens 20 % von der Anstiegskurve bei steigenden Differenzdrücken abweicht. Mehr bevorzugt beträgt die Abweichung höchstens 10 %, meist bevorzugt höchstens 5 %.

Die flexible Wand weist im entspannten Zustand von der gegenüberliegenden Seite einen Abstand von mehr als 1mm und weniger als 3mm, vorzugsweise weniger als 2 mm, und mehr bevorzugt etwa 1,7 mm auf. Über den Abstand wird der maximale Flusswert vorgegeben. Außerdem hat die flexible Wand vorzugsweise eine Dicke von 0,05 bis 0,3 mm, mehr bevorzugt von 0,1 bis 0,2 mm, meist bevorzugt von 0,15 mm. Die Dicke der flexiblen Wand ist somit wesentlich kleiner als die Höhe des Strömungskanals. Die flexible Wand besteht vorzugsweise aus einem elastischen Material und ist mehr bevorzugt eine Silikonmembran oder besteht aus thermoplastischen Elastomeren. Die maximale Länge der flexiblen Wand beträgt vorzugsweise etwa 25 mm, sowie eine maximale Breite von etwa 15 mm.

Gemäß einer weiter bevorzugten Ausführungsform weisen die Einlass- und/oder die Auslassöffnung(en) an den jeweiligen, dem Strömungskanal zugewandten Kanten eine Fase von größer 0,5 und kleiner 1 mm auf. Die Fase kann beispielsweise in Form einer Rundung, etwa in Form eines Viertelkreises, vorzugsweise mit einem Radius von 1 mm vorgesehen sein. Dies ermöglicht erfindungsgemäß eine Verringerung des Druckunterschieds, da der Druck besser in den Strömungskanal verteilt und der Druckgradient verringert wird.

Die Einlassöffnung und die Auslassöffnung sind an gegenüberliegenden Enden des Strömungskanals angeordnet. Vorzugsweise sind die Einlassöffnung und die Auslassöffnung senkrecht zum Strömungskanal angeordnet. Die Einlassöffnung und die Auslassöffnung haben vorzugsweise einen Durchmesser von 5 bis 8 mm, mehr bevorzugt von 6 bis 8 mm, 5 bis 7 mm, oder 6 bis 7 mm, meist bevorzugt von 6,5 mm. Die Mittenachsen der Einlassöffnung und der Auslassöffnung sind vorzugsweise 8 bis 12 mm, mehr bevorzugt 8 bis 11 mm, 8 bis 10 mm, 9 bis 12 mm, 9 bis 11 mm, 10 bis 12 mm, oder 10 bis 11 mm, meist bevorzugt 10 mm voneinander beabstandet.

Wie oben erläutert, weist der Strömungskanal in Ruhe- bzw. Ausgangsstellung in Strömungsrichtung gesehen einen konstanten rechteckigen Querschnitt auf, mit einer großen Breite a im Vergleich zu einer schmalen Höhe b. Bei einer alternativen Ausführungsform ist jedoch der Querschnitt des Strömungskanals in Strömungsrichtung gesehen und ohne anliegenden Differenzdruck, also in Ruhe- bzw. Ausgangsstellung, nicht konstant. Der Strömungskanalquerschnitt weist vielmehr an einer Stelle ein Minimum auf, von dem aus sich der Strömungskanalquerschnitt stromaufwärts und/oder stromabwärts vergrößert. Dieses Minimum liegt bereits im Ruhezustand der Flussbegrenzungsvorrichtung vor, ist also nicht mit einem Minimum vergleichbar bzw. zu verwechseln, das sich ergibt, wenn ein Unterdruck auf den Flussbegrenzer wirkt und sich die Membran wölbt. Besonders bevorzugt liegt ein größer werdender Querschnitt sowohl stromaufwärts als auch stromabwärts vor. Das Minimum liegt dabei vorzugsweise in der Mitte der Länge des Kanals. Doch auch eine außermittige Lage des Minimums ist von der Erfindung als Alternative umfasst. Die Höhe b nimmt also bei dieser Ausführungsform vom Minimum aus zur Einlass- bzw. Auslassöffnung hin zu. Die dem Strömungskanal zugewandte Fläche des ersten und zweiten Gehäuseteils sind in Strömungsrichtung gesehen konvex ausgebildet. Die Erfindung umfasst jedoch ebenso, dass lediglich die Wand des ersten Gehäuseteils, an der die flexible Matte anliegt, konvex ist, während die gegenüberliegende Wand des zweiten Gehäuseteils weiterhin plan ist.

Mit diesem sich aufweitenden Querschnitt des Strömungskanals wird eine nahezu laminare Strömung erreicht und eine höhere Strömungsgeschwindigkeit entsteht. Dadurch wird das Regelverhalten weiter verbessert.

Es ist ferner bevorzugt, dass der Strömungskanalquerschnitt auch quer zur Strömungsrichtung, also in Breitenrichtung a, nicht konstant ist, sondern ein Minimum, vorzugsweise in der Mitte, aufweist und zumindest die Fläche des ersten Gehäuseteils auch in Breitenrichtung konvex ist.

Ferner umfasst die Erfindung ein Inhalations-Handgerät mit einer erfindungsgemäßen Flussbegrenzungsvorrichtung.

Durch die kompakte Bauform der erfindungsgemäßen Flussbegrenzungsvorrichtung kann nun eine Flusskontrolle bei Inhalationssystemen ermöglicht werden, die bisher durch ihre Größe nicht mit derartigen Systemen ausgestattet werden konnten. Die flächig angeordnete flexible Membran und stark verringerte regelnde Fläche ermöglichen sehr kleine Abmessungen des Flussbegrenzungsventils. Gleichzeitig kann durch die neuartige Anordnung der Ein- und Ausströmkanäle eine Flussregelung bei geringen Differenzdrücken erreicht werden. Ein weitere positiver Effekt, der sich durch die geringere Bauform ergibt, ist eine geringere Hysterese der Druck-Flusskurve. Dadurch ist gewährleistet, dass die identischen Flusswerte sowohl bei ansteigendem, als auch bei fallendem Differenzdruck erreicht werden.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem zweiten Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Die erfindungsgemäße Vorrichtung stellt ferner einen Inhalationsfluss von 30 l/min bei einem am Mundstück der Inhalationsvorrichtung anliegendem Differenzdruck von weniger als 30 mbar bereit. Vorzugsweise ist auch ein Fluss von 12 l/min bei weniger als 10mbar, je nach Größe des Strömungskanals möglich.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem dritten Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Ferner ist bei der erfindungsgemäßen Vorrichtung das Verhältnis von regelnder Fläche A des Strömungskanals zu Querschnittsumfang (= 2 x a + 2 x b) des regelnden bzw. aktiven Strömungskanals in Ruhe- bzw. Ausgangsstellung kleiner als 2, bevorzugt kleiner als 1,4.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem vierten Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Ferner ist bei der erfindungsgemäßen Vorrichtung das Verhältnis zwischen Querschnittsfläche von Einsaug- oder Aussaugöffnung zur regelnden Querschnittfläche der flexiblen Wand kleiner 5 zu 1, vorzugsweise kleiner 3 zu 1, und größer 1 zu 1.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem fünften Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Ferner ist bei der erfindungsgemäßen Vorrichtung das Verhältnis von regelnder Fläche A des Strömungskanals zu Querschnitt (d.h. a x b) des Strömungskanals in Ruhestellung, d.h. ohne anliegenden Differenzdruck, kleiner 3, vorzugsweise kleiner 2.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem sechsten Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Die flexible Wand weist im entspannten Zustand von der gegenüberliegenden Seite einen Abstand von mehr als 1 mm und weniger als 3 mm, vorzugsweise weniger als 2 mm, und mehr bevorzugt etwa 1,7 mm auf. Über den Abstand wird der maximale Flusswert vorgegeben.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem siebten Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Ferner ist bei der erfindungsgemäßen Vorrichtung das Verhältnis von regelnder Fläche A des Strömungskanals zu Umfang U der regelnden Fläche in Ruhestellung vorzugsweise kleiner als 2, mehr bevorzugt kleiner als 1 und meist bevorzugt kleiner 0,7.

Die erfindungsgemäße Vorrichtung zur Flussbegrenzung bei niedrigen Differenzdrücken, zur Begrenzung des Inhalationsvolumenstroms bei der Inhalation von therapeutischen Aerosolen, gemäß einem achten Aspekt weist ein Gehäuse mit mindestens einer Einlassöffnung, mindestens einer Auslassöffnung und einem dazwischen befindlichen Strömungskanal auf. Der Strömungskanal ist durch eine sich entlang des Strömungskanals erstreckende flexible Wand begrenzt. Der Strömungskanalquerschnitt weist in Strömungsrichtung gesehen in Ruhe- bzw. Ausgangstellung ein Minimum auf.

Erfindungsgemäß sind die oben im Zusammenhang mit dem ersten Aspekt der Erfindung beschriebenen bevorzugten Ausführungsformen auch einzeln und/oder in Kombination als bevorzugte Ausführungsformen für die beschriebenen zweiten bis achten Aspekte der Erfindung zu verstehen.

Die Erfindung wird nachstehend unter Bezugnahme auf die beigefügten Zeichnungen näher erläutert. Es zeigen:
- Fig. 1a: eine schematische Darstellung einer Draufsicht auf eine erfindungsgemäße Flussbegrenzungsvorrichtung gemäß einer bevorzugten Ausführungsform;
- Fig. 1b: eine Längsschnittdarstellung entlang A-A von Fig. 1a;
- Fig. 1c: eine Querschnittsdarstellung entlang B-B von Fig. 1a;
- Fig. 1d: eine Darstellung der aktiv regelnden Fläche A mit ihrem Umfang U;
- Fig. 2: eine vergleichende Darstellung der Hysteresekurven der erfindungsgemäßen Ausführungsform mit Flussbegrenzern aus dem Stand der Technik;
- Fig. 3-5: die einzelnen Kurvenverläufe aus Fig. 2; und
- Fig.6: eine Längsschnittdarstellung entsprechend Fig. 1b für eine alternative erfindungsgemäße Ausführungsform.

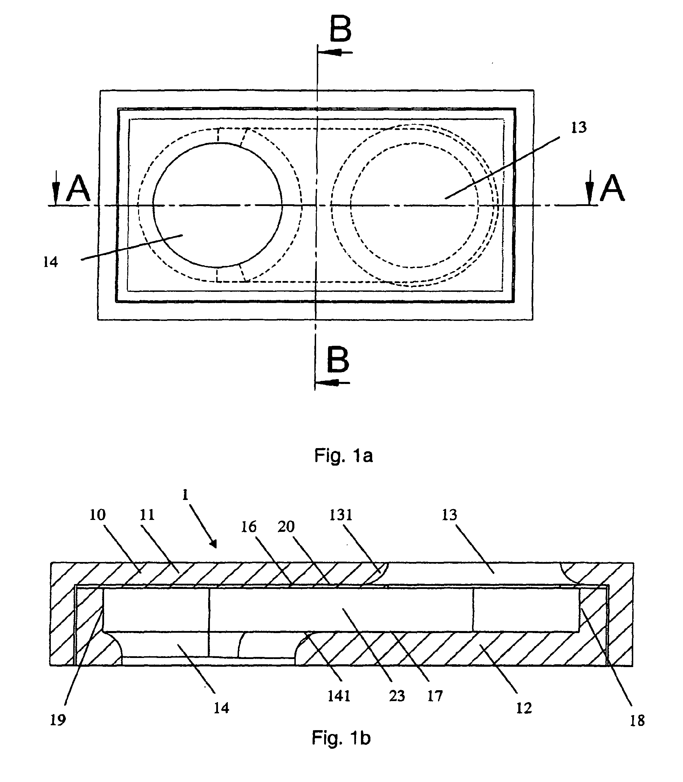

Die Figuren 1a bis 1c zeigen in drei verschiedenen Ansichten eine bevorzugte Ausführungsform der erfindungsgemäßen Flussbegrenzungsvorrichtung. Wie vor allem in Fig. 1b deutlich zu erkennen ist, besteht die erfindungsgemäße Flussbegrenzungsvorrichtung 1 aus einem Gehäuse 10, das eine erste Gehäusehälfte 11 und eine zweite Gehäusehälfte 12 aufweist. Das Gehäuse 10 ist vorzugsweise langgestreckt und beispielsweise quaderförmig und besteht beispielsweise aus Kunststoff. Die erste Gehäusehälfte 11 weist eine Vertiefung auf, in der die zweite Gehäusehälfte 12 eingesetzt ist. Die zweite Gehäusehälfte 12 weist wiederum eine Vertiefung auf, die in zusammengebautem Zustand des Gehäuses 10 einen Strömungskanal 23 bildet. In dem gezeigten Beispiel hat die Vertiefung in der zweiten Gehäusehälfte in etwa die Form einer "0" (in Fig. 1b einer liegenden "0") mit zwei parallel verlaufenden Wänden im mittleren Bereich, die von je einer halbkreisförmigen Wand links und rechts verbunden sind.

Die Zusammensetzung des Gehäuses aus zwei separaten Teilen ist jedoch hier nur beispielhaft gezeigt. Die Erfindung umfasst auch Gehäuseformen, die nicht aus zwei getrennten Teilen bestehen, sondern einstückig aus zwei mit einem Klappmechanismus verbundenen Abschnitten besteht. So sind die beiden Abschnitte beispielsweise in einem Arbeitsgang beispielsweise mit einem Spritzgussverfahren herstellbar. Alternativ dazu kann das Gehäuse des Inhalators bereits einen Teil des Flussbegrenzergehäuses darstellen.

Im ersten Gehäuseteil 11 ist eine Einlassöffnung 13 vorgesehen. Diese ist in der bevorzugten Ausführungsform kreisförmig, wie in Fig. 1a zu erkennen ist. Die Erfindung umfasst jedoch auch Ausführungsformen, in der mehrere Einlassöffnungen vorgesehen sind, sowie Einlassöffnungen anderer Querschnittsformen (beispielsweise oval oder polygonförmig). Das zweite Gehäuseteil 12 weist hingegen eine Auslassöffnung 14 auf. Auch hinsichtlich der Auslassöffnung gilt, dass mehrere Öffnungen vorgesehen sein können, die auch nicht notwendigerweise einen kreisförmigen Querschnitt aufweisen müssen. Bevorzugt ist jedoch sowohl für die Einlassöffnung als auch für die Auslassöffnung ein kreisförmiger Querschnitt. Ferner ist bevorzugt, dass nur genau eine Einlassöffnung und genau eine Auslassöffnung vorgesehen sind.

Zwischen dem ersten Gehäuseteil 11 und dem zweiten Gehäuseteil 12 ist in der bevorzugten Ausführungsform eine flexible Membran beispielsweise aus Silikon oder thermoplastischen Elastomeren 16 eingelegt. Wie in Fig. 1b zu erkennen ist, liegt der in der Figur links dargestellte Teilbereich der Membran 16 flächig an der nach unten zeigenden Wand des ersten Gehäuseteils 11 an. Im Bereich der Einlassöffnung 13 weist auch die Membran 16 eine entsprechende Öffnung auf, um eine Luftströmung von der Einlassöffnung 13 ausgehend, über den Strömungskanal 23, zur Auslassöffnung 14 hin zu ermöglichen. Die flexible Wand kann alternativ auch am Gehäuseteil angespritzt sein, beispielsweise mit einem Zweikomponenen-Spritzgussverfahren. So kann die Membran etwa an eine Stirnseite angespritzt werden.

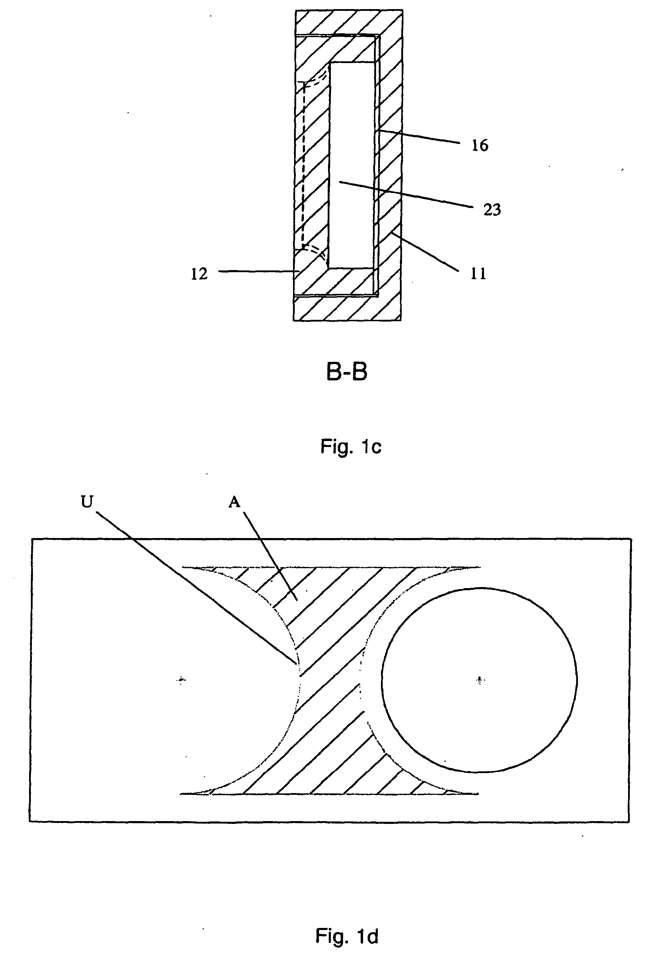

Der Strömungskanal 23 zwischen Einlassöffnung 13 und Auslassöffnung 14 wird somit durch die nach unten weisende Wand 20 der Membran 16 gebildet sowie durch die der Membran 16 gegenüberliegende Wand 17 des zweiten Gehäuseteils 12. Ferner wird der Strömungskanal 23 durch die beiden Seitenwände 18 und 19 begrenzt. Wie in Fig. 1c gezeigt ist, weist der Strömungskanal in Flussrichtung einen rechteckigen Querschnitt auf, mit einer großen Breite a im Vergleich zu einer schmalen Höhe b.

Wird durch die Auslassöffnung 14 Luft angesaugt, strömt diese über die Einlassöffnung 13 in den Strömungskanal 23. Darin entsteht aufgrund des Strömungswiderstandes ein Unterdruck. Dieser Unterdruck im Strömungskanal 23 sorgt dafür, dass sich die Membran 16 nach innen durchbiegt und damit den Querschnitt des Strömungskanals 23 verengt. Diese Teilfläche der Membran 16, die zu einer Verengung des Strömungskanals führt, wird als regelnde Fläche A des erfindungsgemäßen Flussbegrenzers angesehen. Die Durchbiegung der Membran 16 ist umso stärker, je größer der Unterdruck im Strömungskanal 23 ist. Der Querschnitt des Strömungskanals 23 verändert sich somit in Abhängigkeit des Differenzdrucks zwischen Einlassöffnung 13 und Auslassöffnung 14. Da der Volumenstrom wiederum vom Querschnitt des Strömungskanals 23 abhängt, erfolgt über die Querschnittsänderung eine direkte Regelung des Volumenstroms und somit eine Flussbegrenzung.

Die aktiv regelnde Fläche ist in Fig. 1d noch einmal dargestellt, hier schraffiert. Der Umfang U der aktiv regelnden Fläche setzt sich aus den beiden parallelen geraden Teilstrecken, sowie den beiden gegenüberliegenden Kreissegmenten zusammen.

Durch die degressive Materialflexibilität nimmt dabei die für die Durchbiegung der Membran erforderliche Kraft mit zunehmendem Unterdruck im Strömungskanal bis zu einem Grenzwert zu, der den gewünschten minimalen Strömungskanalquerschnitt zur Begrenzung des Volumenstromes bestimmt.

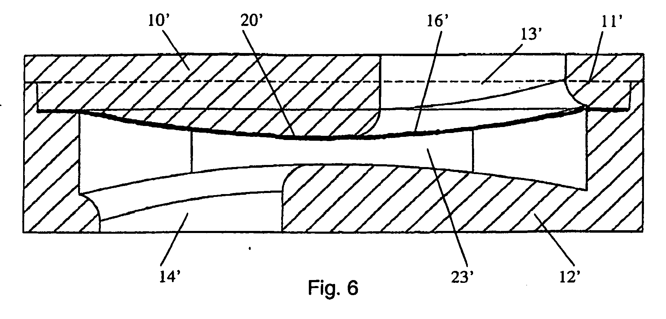

Fig. 6 zeigt eine Schnittansicht einer anderen bevorzugten Ausführungsform der erfindungsgemäßen Flussbegrenzungsvorrichtung. Diese Flussbegrenzungsvorrichtung 1' weist ein Gehäuse 10' auf, das eine erste Gehäusehälfte 11' und eine zweite Gehäusehälfte 12' hat. Das Gehäuse ist langgestreckt und beispielsweise quaderförmig. Es besteht beispielsweise aus Kunststoff. Die erste Gehäusehälfte 11' weist eine Vertiefung auf, in der die zweite Gehäusehälfte 12' aufnehmbar ist bzw. eingesetzt ist. Die zweite Gehäusehälfte 12' weist wiederum eine Vertiefung auf, die in zusammengebautem Zustand des Gehäuses 10' einen Strömungskanal 23' bildet. In dem gezeigten Beispiel hat die Vertiefung wie in Fig. 1a für die vorstehend beschriebene Ausführungsform zu erkennen in der zweiten Gehäusehälfte 12' in etwa die Form einer "0" (in Fig. 1b einer liegenden "0") mit zwei parallel verlaufenden Wänden im mittleren Bereich, die von je einer halbkreisförmigen Wand links und rechts verbunden sind.

Auch für diese Ausführungsform ist die Zusammensetzung des Gehäuses aus zwei separaten Teilen nur beispielhaft gezeigt. Die Erfindung umfasst auch Gehäuseformen, die nicht aus zwei getrennten Teilen bestehen, sondern einstückig aus zwei mit einem Klappmechanismus verbundenen Abschnitten besteht. So sind die beiden Abschnitte beispielsweise in einem Arbeitsgang beispielsweise mit einem Spritzgussverfahren herstellbar. Alternativ dazu kann das Gehäuse des Inhalators bereits einen Teil des Flussbegrenzergehäuses darstellen.

Im ersten Gehäuseteil 11' ist eine Einlassöffnung 13' vorgesehen. Diese ist beispielsweise kreisförmig. Es sind jedoch auch Ausführungsformen umfasst, in denen mehrere Einlassöffnungen vorgesehen sind, sowie Einlassöffnungen anderer Querschnittsformen (beispielsweise oval oder polygonförmig). Das zweite Gehäuseteil 12' weist hingegen eine Auslassöffnung 14' auf. Auch hinsichtlich der Auslassöffnung gilt, dass mehrere Öffnungen vorgesehen sein können, die auch nicht notwendigerweise einen kreisförmigen Querschnitt aufweisen müssen. Bevorzugt ist jedoch sowohl für die Einlassöffnung als auch für die Auslassöffnung ein kreisförmiger Querschnitt. Ferner ist bevorzugt, dass nur genau eine Einlassöffnung und genau eine Auslassöffnung vorgesehen sind.

Zwischen dem ersten Gehäuseteil 11' und dem zweiten Gehäuseteil 12' ist in der bevorzugten Ausführungsform von Fig. 6 eine flexible Silikonmatte 16' eingelegt. Wie in Fig. 6 zu erkennen ist, liegt der in der Figur links dargestellte Teilbereich der Membran 16' flächig an der nach unten zeigenden Wand des ersten Gehäuseteils 11' an. Im Bereich der Einlassöffnung 13' weist auch die Membran 16' eine entsprechende Öffnung auf, um eine Luftströmung von der Einlassöffnung 13' ausgehend, über den Strömungskanal 23', zur Auslassöffnung 14' hin zu ermöglichen. Auch in dieser Ausführungsform kann die flexible Wand, wie oben erläutert, angespritzt sein.

Der Strömungskanal 23' zwischen Einlassöffnung 13' und Auslassöffnung 14' wird somit durch die nach unten weisende Wand 20' der Membran 16' gebildet sowie durch die der Membran 16' gegenüberliegende Wand des zweiten Gehäuseteils 12'. Ferner wird der Strömungskanal 23' durch die beiden Seitenwände 18' und 19' begrenzt.

Wie in Fig. 1c gezeigt ist, weist auch der Strömungskanal von Fig. 6 in Flussrichtung grundsätzlich einen rechteckigen Querschnitt auf, mit einer großen Breite a im Vergleich zu einer schmalen Höhe b. Bei der Ausführungsform gemäß Fig. 6 ist jedoch der Querschnitt des Strömungskanals in Ruhestellung in Strömungsrichtung gesehen nicht konstant. Der Strömungskanalquerschnitt weist vielmehr an einer Stelle ein Minimum auf, von dem aus sich der Strömungskanalquerschnitt stromaufwärts und/oder stromabwärts vergrößert. In dem in Fig. 6 gezeigten Beispiel liegt ein größer werdender Querschnitt sowohl stromaufwärts als auch stromabwärts vor. Das Minimum liegt dabei in der Mitte der Länge des Kanals. Doch auch eine außermittige Lage des Minimums ist von der Erfindung als Alternative umfasst. Anders ausgedrückt nimmt die Höhe b vom Minimum aus zur Einlass- bzw. Auslassöffnung hin zu. Die dem Strömungskanal zugewandte Fläche des ersten und zweiten Gehäuseteils 11' bzw. 12' sind wie in Fig. 6 zu sehen konvex ausgebildet.

Wird durch die Auslassöffnung 14' Luft angesaugt, strömt diese über die Einlassöffnung 13' in den Strömungskanal 23'. Darin entsteht aufgrund des Strömungswiderstandes ein Unterdruck. Dieser Unterdruck im Strömungskanal 23' sorgt dafür, dass sich die Membran 16' nach innen durchbiegt und damit den Querschnitt des Strömungskanals 23' verengt. Diese Teilfläche der Membran 16', die zu einer Verengung des Strömungskanals führt, wird als regelnde Fläche des erfindungsgemäßen Flussbegrenzers angesehen. Die Durchbiegung der Membran 16' ist umso stärker, je größer der Unterdruck im Strömungskanal 23' ist. Der Querschnitt des Strömungskanals 23' verändert sich somit in Abhängigkeit des Differenzdrucks zwischen Einlassöffnung 13' und Auslassöffnung 14'. Da der Volumenstrom wiederum vom Querschnitt des Strömungskanals 23' abhängt, erfolgt über die Querschnittsänderung eine direkte Regelung des Volumenstroms und somit eine Flussbegrenzung.

Durch die degressive Materialflexibilität nimmt dabei die für die Durchbiegung der Membran erforderliche Kraft mit zunehmendem Unterdruck im Strömungskanal bis zu einem Grenzwert zu, der den gewünschten minimalen Strömungskanalquerschnitt zur Begrenzung des Volumenstromes bestimmt.

Die erfindungsgemäße Flussbegrenzungsvorrichtung ist im Vergleich zu bekannten Flussbegrenzungsvorrichtungen aus dem Stand der Technik deutlich in seinen Abmessungen kleiner. So ist beispielsweise die erfindungsgemäße Flussbegrenzungsvorrichtung gegenüber der Flussbegrenzung aus der DE-A-100 29 119 um ca. den Faktor 5 verkleinert. Erfindungsgemäß wurde jedoch die Flussbegrenzungsvorrichtung nicht lediglich in ihren Abmessungen verkleinert (downscaling), sondern vielmehr hinsichtlich verschiedenster Parameter neu gestaltet, um überhaupt die Funktionsfähigkeit bei dieser deutlichen Verkleinerung beizubehalten. Eine bloße Verkleinerung der bekannten Flussbegrenzungsvorrichtung würde nicht zu einer funktionsfähigen Flussbegrenzung führen.

Die folgende Tabelle vergleicht ein Ausführungsbeispiel der erfindungsgemäßen Flussbegrenzungsvorrichtung gemäß Fig. 1a-1c (rechte Spalte) mit zwei bekannten Vorrichtungen. Die aus der DE 199 12 461 bekannte Flussbegrenzungsvorrichtung wird beispielsweise in den Inhalationsgeräteprototypen der Firma Activaero GmbH, Gemünden, Deutschland verwendet, und die aus der DE 100 29 119 bekannte Flussbegrenzungsvorrichtung ist als Ventil LimiX™ der Firma Activaero GmbH, Gemünden, Deutschland, bekannt und wird beispielsweise in den Inhalationsgeräten der Serie Watchhaler^{™} der Firma Activaero GmbH, Gemünden, Deutschland verwendet.

| **Flächen in mm²** | **DE 199 12 461** | **DE 100 29 119** | **Erfindungsgemäßes Beispiel** |
|---|---|---|---|
| Einlassöffnung | 78,53 | 28,27 | 33,18 |
| Auslassöffnung | 78,53 | 56,54 | 33,18 |
| Grundfläche Membran | 8320 | 1290 | 200 |
| Grundfläche variabler Strömungskanal | 4013,13 | 584,41 | 77,24 |
| regelnde Fläche A des Strömungskanals | 3934,6 | 556,14 | 26,98 |
| Querschnitt Strömungskanal in Ruhestellung (ohne Differenzdruck) | 40 | 98,23 | 13,6 |

### Umfang in mm

| | | | |
|---|---|---|---|
| Umfang Membran in mm | 424 | 127,23 | 60 |
| Umfang U der regelnden Fläche | 805,96 | 236,73 | 39,58 |
| Querschnittsumfang Strömungskanal in Ruhestellung (ohne Differenzdruck) | 82 | 85,96 | 19,4 |

### Verhältnisse

| | | | |
|---|---|---|---|
| Querschnitt Strömungskanal/ Querschnittsumfang Strömungskanal in Ruhestellung | 0,44 | 0,46 | 0,70 |
| regelnde Fläche A des Strömungskanals / Querschnitt Strömungskanal in Ruhestellung | 98,37 | 5,56 | 1,98 |
| regelnde Fläche A des Strömungskanals / Umfang U der regelnden Fläche | 4,88 | 2,35 | 0,68 |

Erfindungsgemäß weist die Flussbegrenzungsvorrichtung eine regelnde Fläche von weniger als 100 mm² auf, in dem in der Tabelle gezeigten Beispiel von lediglich etwa 26,98 mm².

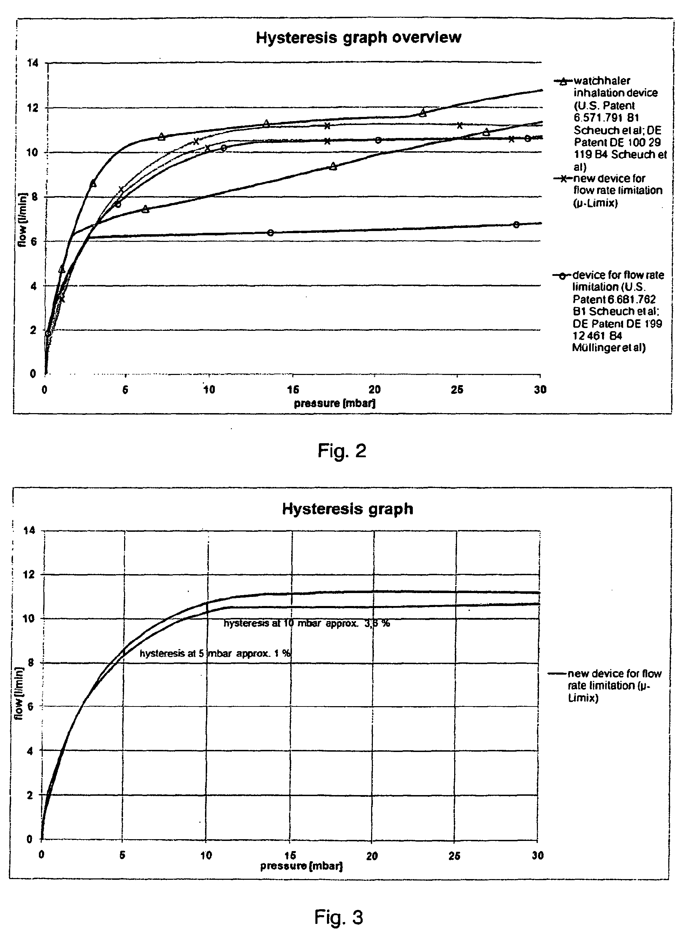

Vor allem in Kombination der Parameter "regelnde Fläche", das Verhältnis von regelnder Fläche zum Umfang des Strömungskanals in Ruhestellung und Abschrägung der Kanten der Ein- und Auslassöffnungen ergibt sich eine deutlich verbesserte Funktionsweise gegenüber bekannten Flussbegrenzungsvorrichtungen, und dies trotz der deutlich verkleinerten Bauart. Dies wird anhand der Figuren 2 bis 5 deutlich.

Fig. 3 zeigt die Hysteresekurve, die das Flussbegrenzungsverhalten des erfindungsgemäßen Flussbegrenzers nach Figuren 1a bis 1c widerspiegelt. In Fig. 3, wie auch in den Figuren 2, 4 und 5, ist hierbei lediglich ein Bereich von 0 bis 30 mbar dargestellt, da dies der für die erfindungsgemäße Flussbegrenzungsvorrichtung relevante Differenzdruckbereich ist. Fig. 3 zeigt deutlich, dass für die erfindungsgemäße Flussbegrenzungsvorrichtung eine annähernd ideale, sehr flache Hysteresekurve erreicht wird. Bei einem Differenzdruck von 5 mbar weicht die Anstiegskurve um lediglich 1 % von der fallenden Kurve ab. Bei einem Differenzdruck von 10 mbar ist der Unterschied lediglich 3,6 %.

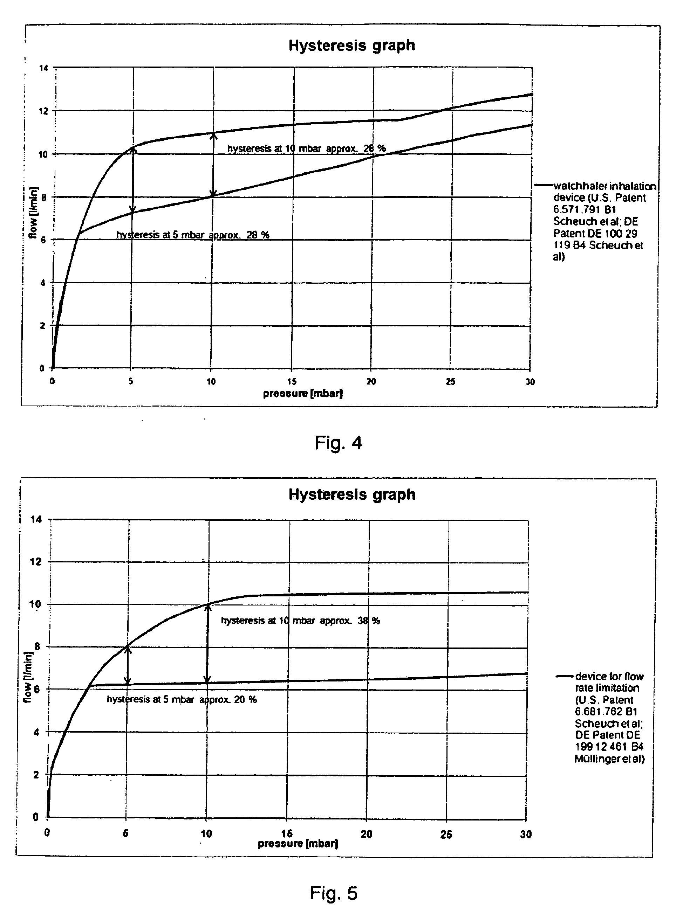

Im Vergleich dazu ist in Fig. 4 das Flussbegrenzungsverhalten des aus der DE-A-100 29 119 bekannten Flussbegrenzers dargestellt. In Fig. 4 ist die deutlich ausgeprägtere Hysterese zu erkennen, bei der sich sowohl bei einem Differenzdruck von 5 mbar als auch bei 10 mbar ein Unterschied von etwa 28 % zwischen ansteigender und abfallender Druckkurve ergibt.

Fig. 5 zeigt die Hysterese für den aus der DE-A-199 12 461 bekannten Flussbegrenzer. Auch hier ist die Hysterese deutlich ausgeprägt mit einem Unterschied von 20 % bei einem Differenzdruck von 5 mbar bzw. einem Unterschied von 38 % bei einem Differenzdruck von 10 mbar.

Zur besseren Vergleichbarkeit sind die Graphen der Fig. 3 bis 5 noch einmal gemeinsam in Fig. 2 dargestellt.

Im Vergleich zu den bekannten Flussbegrenzem wird somit mit der erfindungsgemäßen Flussbegrenzungsvorrichtung ein nahezu ideales Flussbegrenzungsverhalten erzielt.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren auch wenn sie dort im Zusammenhang mit anderen Merkmalen gezeigt sind und/oder vorstehend nicht genannt sind.

Im Weiteren schließt der Ausdruck "umfassen" und Ableitungen davon andere Elemente oder Schritte nicht aus. Ebenfalls schließt der unbestimmte Artikel "ein" bzw. "eine" und Ableitungen davon eine Vielzahl nicht aus. Die Funktionen mehrerer in den Ansprüchen aufgeführter Merkmale können durch eine Einheit erfüllt sein. Die bloße Tatsache, dass bestimmte Masse in zueinander verschiedenen abhängigen Ansprüchen aufgeführt sind, bedeutet nicht, dass eine Kombination dieser Masse nicht vorteilhaft verwendet werden kann. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

## Patentansprüche

1. Vorrichtung zur Flussbegrenzung bei niedrigen Differenzdrücken, zur Begrenzung des Inhalationsvolumenstroms bei der Inhalation von therapeutischen Aerosolen, mit einem Gehäuse (10, 10') mit mindestens einer Einlassöffnung (13, 13'), mindestens einer Auslassöffnung (14, 14') und einem dazwischen befindlichen Strömungskanal (23, 23'), wobei der Strömungskanal (23, 23') durch eine sich entlang des Strömungskanals (23, 23') erstreckende flexible Wand (16, 16') begrenzt ist, **dadurch gekennzeichnet, dass** die flexible Wand (16, 16') eine regelnde Fläche A von weniger als 100mm² aufweist und dass die kürzeste Länge des Strömungskanals (23, 23') kleiner als 5 mm ist.

2. Vorrichtung nach Anspruch 1, wobei der Strömungskanal (23, 23') einen flachen länglichen Querschnitt aufweist, der durch die flexible Wand (16, 16'), eine der flexiblen Wand gegenüberliegende Wand (17, 17'), und zwei Querwände (18, 19; 18', 19') gebildet ist.

3. Vorrichtung nach Anspruch 2, wobei die Vorrichtung mindestens eine flexible Wand (16, 16') aufweist, wobei die mindestens eine flexible Wand (16, 16') den Querschnitt des Strömungskanals (23, 23') durch den bei einer Inhalation des Aerosols entstehenden Unterdruck reduziert.

4. Vorrichtung nach einem der vorstehenden Ansprüche, wobei die regelnde Fläche A kleiner als 75mm², vorzugsweise kleiner als 50 mm² ist.

5. Vorrichtung nach einem der vorstehenden Ansprüche, wobei das Verhältnis von regelnder Querschnittsfläche A in mm² zu Querschnittsumfang in mm des Strömungskanals (23, 23') in Ruhestellung kleiner 2, vorzugsweise kleiner 1,4, ist.

6. Vorrichtung nach einem der vorstehenden Ansprüche, wobei das Verhältnis zwischen Einlass- oder Auslassöffnungsquerschnitt in mm² zur regelnden Fläche A in mm² kleiner 5 zu 1, vorzugsweise kleiner 3 zu 1, und größer 1:1 ist.

7. Vorrichtung nach einem der vorstehenden Ansprüche, wobei das Verhältnis von regelnder Fläche A in mm² des variablen Strömungskanals (23, 23') zum Strömungskanal (23, 23') Querschnitt in mm² in Ruhestellung kleiner 2 ist.

8. Vorrichtung nach einem der vorstehenden Ansprüche, wobei das Flussbegrenzungsverhalten bis zu einem Differenzdruck von 30 mbar eine Hysteresekurve aufweist, die bei fallenden Differenzdruckwerten höchstens 20 % von der Anstiegskurve bei steigenden Differenzdrücken abweicht.

9. Vorrichtung nach Anspruch 8, wobei die Abweichung höchstens 10 %, vorzugsweise höchstens 5 % beträgt.

10. Vorrichtung nach einem der vorstehenden Ansprüche, wobei die flexible Wand (16, 16') im entspannten Zustand von der gegenüberliegenden Seite einen Abstand von mehr als 1mm und weniger als 3mm aufweist.

11. Vorrichtung nach einem der vorstehenden Ansprüche, wobei die flexible Wand (16, 16') eine Dicke von weniger als 0,3 mm aufweist.

12. Vorrichtung nach einem der vorstehenden Ansprüche, wobei die Einlass- und/oder Auslassöffnungen (13, 14; 13', 14') an den jeweiligen, dem Strömungskanal (23, 23') zugewandten Kanten eine Fase (131, 141) von größer 0,5 und kleiner 1 mm aufweisen.

13. Vorrichtung nach einem der vorstehenden Ansprüche, wobei die Einlassöffnung (13, 13') und die Auslassöffnung (14, 14') an gegenüberliegenden Enden des Strömungskanals (23, 23') angeordnet sind und/oder wobei die Einlassöffnung (13, 13') und die Auslassöffnung (14, 14') senkrecht zum Strömungskanal (23, 23') angeordnet sind.

14. Vorrichtung nach einem der vorstehenden Ansprüche, wobei der Strömungskanalquerschnitt in Ruhestellung in Strömungsrichtung gesehen ein Minimum hat.

15. Inhalations-Handgerät mit einer Flussbegrenzungsvorrichtung (1, 1') nach einem der vorstehenden Ansprüche.

## Claims

1. A device for a flow rate limitation at low differential pressures, for the limitation of the inhalation volume flow during the inhalation of therapeutic aerosols, comprising a housing (10, 10') with at least one inlet opening (13, 13'), at least one outlet opening (14, 14') and a flow channel (23, 23') arranged therebetween, wherein the flow channel (23, 23') is restricted by a flexible wall (16, 16') extending along the flow channel (23, 23'), **characterised in that** the flexible wall (16, 16') has a control area A of less than 100 mm² and the shortest length of the flow channel (23, 23') is smaller than 5 mm.

2. The device according to claim 1, wherein the flow channel (23, 23') has a planar elongate cross-section, formed by the flexible wall (16, 16'), a wall (17, 17') opposing the flexible wall, and two cross walls (18, 19; 18', 19').

3. The device according to claim 2, wherein the device comprises at least one flexible wall (16, 16'), wherein the at least one flexible wall (16, 16') reduces the cross-section of the flow channel (23, 23') by the negative pressure created by the inhalation of aerosol.

4. The device according to any one of the preceding claims, wherein the control area A is smaller than 75 mm², preferably smaller than 50 mm².

5. The device according to any one of the preceding claims, wherein the ratio of control cross-sectional area A in mm² to cross-section periphery of the flow channel (23, 23') in neutral state in mm is less than 2, preferably less than 1.4.

6. The device according to any one of the preceding claims, wherein the ratio in mm² between the cross-section of the inlet or outlet opening to control area A in mm² is less than 5 to 1, preferably less than 3 to 1, and greater 1:1.

7. The device according to any one of the preceding claims, wherein the ratio of control cross-sectional area A of the variable flow channel (23, 23') in mm² to flow-channel (23, 23') cross-section in mm² in neutral state is less than 2.

8. The device according to any one of the preceding claims, wherein the flow rate limitation behaviour up to a differential pressure of 30 mbar exhibits a hysteresis graph which, at falling differential pressure values, deviates no more than 20% from the growth curve at rising differential pressures.

9. The device according to claim 8, wherein the deviation is no more than 10%, preferably no more than 5%.

10. The device according to any of the preceding claims, wherein, in unstressed condition, the flexible wall (16, 16') has a distance of more than 1 mm and less than 3 mm from the opposite side.

11. The device according to any of the preceding claims, wherein the flexible wall (16, 16') has a thickness of less than 0.3 mm.

12. The device according to any one of the preceding claim, wherein the inlet and/or outlet openings (13, 14; 13', 14') have a chamfer (131, 141) of more than 0.5 and less than 1 mm at the respective edges facing the flow channel (23, 23').

13. The device according to any one of the preceding claims, wherein the inlet opening (13, 13') and the outlet opening (14, 14') are arranged at opposite ends of the flow channel (23, 23') and/or wherein the inlet opening (13, 13') and the outlet opening (14, 14') are arranged perpendicularly to the flow channel (23, 23').

14. The device according to any of the preceding claims; wherein the flow channel cross-section in neutral state has a minimum in the flow direction.

15. A hand-held inhalation device with a flow rate limitation device (1, 1') according to any one of the preceding claims.

## Revendications

1. Dispositif de limitation de flux à basses pressions différentielles, pour limiter le débit volumique d'inhalation lors de l'inhalation d'aérosols thérapeutiques, comportant un boîtier (10, 10') pourvu d'au moins un orifice d'entrée (13, 13'), d'au moins un orifice de sortie (14, 14') et d'un canal d'écoulement (23, 23') situé entre ces orifices, le canal d'écoulement (23, 23') étant délimité par une paroi souple (16, 16') s'étendant le long du canal d'écoulement (23, 23'), **caractérisé en ce que** la paroi souple (16, 16') comporte une surface réglante A inférieure à 100 mm², et **en ce que** la longueur la plus courte du canal d'écoulement (23, 23') est inférieure à 5 mm.

2. Dispositif selon la revendication 1, dans lequel le canal d'écoulement (23, 23') a une section oblongue plate qui est formée par la paroi souple (16, 16'), par une paroi (17, 17') opposée à la paroi souple et par deux parois transversales (18, 19 ; 18', 19').

3. Dispositif selon la revendication 2, dans lequel le dispositif comporte au moins une paroi souple (16, 16'), la au moins une paroi souple (16, 16') réduisant la section du canal d'écoulement (23, 23') par la dépression créée lors d'une inhalation de l'aérosol.

4. Dispositif selon l'une quelconque des revendications précédentes, dans lequel la surface réglante A est inférieure à 75 mm², de préférence inférieure à 50 mm².

5. Dispositif selon l'une quelconque des revendications précédentes, dans lequel le rapport de l'aire de section réglante A en mm² sur la circonférence de section en mm du canal d'écoulement (23, 23') en position de repos est inférieur à 2, de préférence inférieur à 1,4.

6. Dispositif selon l'une quelconque des revendications précédentes, le rapport entre la section de l'orifice d'entrée ou de sortie en mm² et la surface réglante A en mm² est inférieur à 5 sur 1, de préférence inférieur à 3 sur 1, et supérieur à 1:1.

7. Dispositif selon l'une quelconque des revendications précédentes, dans lequel le rapport de la surface réglante A en mm² du canal d'écoulement (23, 23') variable sur la section du canal d'écoulement (23, 23') en mm² en position de repos est inférieur à 2.

8. Dispositif selon l'une quelconque des revendications précédentes, dans lequel le comportement de limitation de flux jusqu'à une pression différentielle de 30 mbar présente une courbe d'hystérésis qui, avec des valeurs de pression différentielle décroissantes, s'écarte au plus de 20 % de la courbe de croissance avec des pressions différentielles croissantes.

9. Dispositif selon la revendication 8, dans lequel l'écart est au plus de 10 %, de préférence au plus de 5 %.

10. Dispositif selon l'une quelconque des revendications précédentes, dans lequel la paroi souple (16, 16') se trouve, en l'absence de sollicitation, à une distance du côté opposé de plus de 1 mm et de moins de 3 mm.

11. Dispositif selon l'une quelconque des revendications précédentes, dans lequel la paroi souple (16, 16') a une épaisseur inférieure à 0,3 mm.

12. Dispositif selon l'une quelconque des revendications précédentes, dans lequel les orifices d'entrée et/ou de sortie (13, 14 ; 13', 14') comportent sur les bords respectifs tournés vers le canal d'écoulement (23, 23') un chanfrein (131, 141) supérieur à 0,5 et inférieur à 1 mm.

13. Dispositif selon l'une quelconque des revendications précédentes, dans lequel l'orifice d'entrée (13, 13') et l'orifice de sortie (14, 14') sont disposés à des extrémités opposées du canal d'écoulement (23, 23') et/ou l'orifice d'entrée (13, 13') et l'orifice de sortie (14, 14') sont disposés perpendiculairement au canal d'écoulement (23, 23').

14. Dispositif selon l'une quelconque des revendications précédentes, dans lequel la section du canal d'écoulement en position de repos a un minimum, considéré dans la direction d'écoulement.

15. Appareil d'inhalation à main avec un dispositif de limitation de flux (1, 1') selon l'une quelconque des revendications précédentes.
